# EUROPÄISCHE PATENTSCHRIFT

(11) **EP 2 777 723 B1**
(45) Veröffentlichungstag und Bekanntmachung des Hinweises auf die Patenterteilung: **20.12.2017**
(21) Anmeldenummer: 14152461.1
(22) Anmeldetag: 24.01.2014
(51) Int. Cl.: A61L 27/34, A61L 27/58, A61L 29/08, A61L 29/14, A61L 31/10, A61L 31/14

(54) **Implantierbarer Gegenstand umfassend gezielt degradierbare Co-Polymere zur verbesserten Explantierbarkeit**
Implantable object comprising selectively degradable copolymers for improved explantability
Objet pouvant être implanté et contenant des copolymères dégradables de façon ciblée pour améliorer la capacité d'explantation

(30) Priorität: 14.03.2013 US 201361781023 P
(43) Veröffentlichungstag der Anmeldung: 17.09.2014
(73) Patentinhaber: Biotronik AG, 8180 Bülach (CH)
(72) Erfinder: Ulmer, Jens, 8700 Küsnacht (CH); Diebold, Michael, 12169 Berlin (DE)
(74) Vertreter: Randoll, Sören

(56) Entgegenhaltungen:
- US-A- 5 902 329
- US-A1- 2011 027 181
- US-A1- 2011 238 163
- HUANGHAO YANG ET AL: "Engineering Target-Responsive Hydrogels Based on Aptamer-Target Interactions", JOURNAL OF THE AMERICAN CHEMICAL SOCIETY, Bd. 130, Nr. 20, 29. April 2008 (2008-04-29), Seiten 6320-6321, XP055156555, ISSN: 0002-7863, DOI: 10.1021/ja801339w
- PARTHA RAY ET AL: "Application of Aptamers for Targeted Therapeutics", ARCHIVUM IMMUNOLOGIAE ET THERAPIAE EXPERIMENTALIS, Bd. 61, Nr. 4, 7. April 2013 (2013-04-07), Seiten 255-271, XP055156603, ISSN: 0004-069X, DOI: 10.1007/s00005-013-0227-0

### SEQUENCE LISTING

<110> BIOTRONIK AG
<120> Implantierbarer Gegenstand umfassend gezielt degradierbare Co-Polymere zur verbesserten Explantierbarkeit
<130> 10.255P-EP
<140> 14152461.1
   <141> 24.01.2014
<150> US61/781,023
   <151> 2013-03-14
<160> 6
<170> PatentIn version 3.5
<210> 1
   <211> 11
   <212> DNA
   <213> artificial sequence
<220>
   <223> synthetic sonstruct
<400> 1
   auaccauaug c 11
<210> 2
   <211> 11
   <212> DNA
   <213> artificial sequence
<220>
   <223> synthetic construct
<400> 2
   uaugguauac g 11
<210> 3
   <211> 15
   <212> DNA
   <213> artificial sequence
<220>
   <223> synthetic construct
<220>
   <221> misc_feature
   <222> (1)..(1)
   <223> 5' acrydite
<400> 3
   aaaaauacca uaugc 15
<210> 4
   <211> 15
   <212> DNA
   <213> artificial sequence
<220>
   <223> synthetic construct
<220>
   <221> misc_feature
   <222> (1)..(1)
   <223> 5' acrydite
<400> 4
   aaaauauggu auacg 15
<210> 5
   <211> 21
   <212> DNA
   <213> artificial sequence
<220>
   <223> sythetic construct
<220>
   <221> misc_feature
   <222> (1)..(1)
   <223> 5' acrydite
<220>
   <221> misc_feature
   <222> (21)..(21)
   <223> 3' acrydite
<400> 5
   aaaagccgua gguugcccaa a 21
<210> 6
   <211> 61
   <212> DNA
   <213> artificial sequence
<220>
   <223> synthetic construct
<220>
   <221> misc_feature
   <222> (1)..(1)
   <223> 5' poly(p-phenylene)
<400> 6
   gggcgacccu gaugagucug gauaccaugc augaugcacc uuggcagucu uacgaaacgg 60
   u 61

## Beschreibung

Die Erfindung betrifft einen implantierbaren Gegenstand, umfassend eine Schicht, umfassend ein Co-Polymer mit einer Aptameruntereinheit, wobei in Anhängigkeit von einem Bindungsereignis an das Aptamer Bindungen im Co-Polymer gelöst werden können, so dass eine Degradation des Co-Polymers erfolgt. Dies ermöglicht eine verbesserte Explantierbarkeit. Die Erfindung betrifft insbesondere implantierbare Gegenstände, bei denen die Co-Polymere so eingerichtet sind, dass sie bei Auftreten eines vorbestimmten Stimulus, der körpereigen oder körperfremd sein kann, im implantierten Zustand degradiert werden.

Die Degradation von Implantaten oder Teilen von Implantaten, hier insbesondere von Beschichtungen ist ein Vorgang, dem aus medizinischen Gründen viel Aufmerksamkeit gewidmet wird. Zum einen ist eine Kontrolle der Abbaugeschwindigkeit von Implantaten im Körper wichtig, um zu gewährleisten, dass die Implantate für eine ausreichend lange Zeit ihre Funktion behalten. Im Extremfall ist also eine Degradation komplett unerwünscht. Andererseits führt eine Vielzahl von Materialien im Körper zu Unverträglichkeitsreaktionen. Diesem kann entgegnet werden, indem die Implantate mit Beschichtungen überzogen werden. Im Stand der Technik sind Beschichtungen bekannt, die das Einwachsverhalten und damit die Akzeptanz des Implantates im Körper positiv beeinflussen, indem sie einem kontrollierten Abbau durch das körpereigene Milieu unterliegen.

Es kann auch erwünscht sein, dass Implantate komplett im Körper abgebaut werden, z. B. weil ein Stent nach einer ausreichenden Zeit mechanischer Unterstützung des jeweiligen Gefäßes aufgrund der Körperreaktionen nicht mehr benötigt wird.

Ein weiterer Ansatz, bei dem es wünschenswert ist, dass Teile von Implantaten degradiert werden, ist folgender: Die äußeren Schichten eines Implantates werden mit Wirkstoffen beladen, die primär nicht durch Diffusion freigesetzt werden, sondern durch die Degradation der Wirkstoff tragenden Schicht. Damit ist ein verzögertes Freisetzen des Wirkstoffes möglich. Vor diesem Hintergrund ist es interessant, das Degradationsverhalten von Implantaten bzw. von Teilen der Implantate möglichst genau zu kontrollieren.

Es existieren Polymere, die unter physiologischen Bedingungen ein konstantes Abbauverhalten aufweisen. So zeigen z.B. Polyester, Polyorthoester, Polyalkohole, Polyether, Polyamide, Polyurethane und Polyanhydride unter Einfluss von Enzymen (wie z. B. Esterasen, Lipasen, Dehydrogenasen oder Oxygenasen) eine konstante Degradationsgeschwindigkeit. Weiterhin unterliegen die meisten dieser Polymere der allgemeinen Hydrolyse unter physiologischen Bedingungen und werden somit nach Kontakt mit Wasser über einen längeren Zeitraum hin abgebaut. Diese Polymere lassen sich durch ihre molekulare Zusammensetzung (Verwendung von Co-Polymeren, Polymermischungen, Art und Grad der Vernetzung) in Ihrer Degradationsgeschwindigkeit steuern, indem man die Anteile an hydrolyseempfindlichen Bindungseinheiten mit nichtdegradierbaren Einheiten kombiniert, bzw. hydrophile und hydrophobe Anteile variiert. Damit lassen sich konstante Degradationsgeschwindigkeiten von wenigen Tagen bis mehreren Monaten verwirklichen.

Ein konstantes Degradationsverhalten ist aber in vielen Fällen eine unzureichende Lösung: Es ist wünschenswert, dass eine möglichst differenzierte Steuerung der Degradation insbesondere von Polymerbeschichtungen auf Implantaten erfolgt. Dies gilt insbesondere für den Fall, dass ein Implantat wieder aus dem Körper entfernt werden soll: Insbesondere bei Implantaten, die längere Zeit im Körper verblieben sind, kommt es regelmäßig zu Einwachsprozessen. Dies ist meistens erwünscht, da so eine Stabilität des Implantates im Körper gewährleistet wird. Daher ist es spätestens zu dem Zeitpunkt, zu dem der Einwachsprozess abgeschlossen ist, wünschenswert, dass ein Implantat eine stabile Oberfläche besitzt.

In vielen Fällen ist es aber wünschenswert oder sogar erforderlich, ein Implantat wieder aus dem Körper zu entfernen. Grund hierfür kann beispielsweise sein, dass das Implantat seine Funktion erfüllt hat und nicht weiter benötigt wird oder aber dass das Implantat beschädigt ist und ausgetauscht werden muss. Ein typisches Beispiel für einen letzteren Fall ist ein Elektronenbruch bei Herzschrittmachern. Durch das - an sich erwünschte - Einwachsen des Implantates in den Körper ist eine Explantation aber wesentlich erschwert. Durch den festen Kontakt zwischen körpereigenem Gewebe und Implantatoberfläche kommt es bei der Explantation häufig zu erheblichen Gewebeverletzungen. Dementsprechend ist es wünschenswert, dass eine Beschichtung eines Implantates erst dann degradiert, wenn das Implantat wieder aus dem Körper entnommen werden soll. Hierzu existieren im Stand der Technik Polymere die durch gezielte externe Beeinflussung das Abbauverhalten verändern. Zu diesen Einflüssen gehören unter anderem Änderungen von pH, Ionenstärke und Temperatur. Auch Bestrahlung mit elektromagnetischer Energie und sonstigen ionisierenden Strahlen bzw. Einwirkung von elektrischen oder magnetischen Feldern können ebenfalls zum Auslösen der Degradation herangezogen werden.

So haben zum Beispiel Polymere aus der Gruppe von Acrylamid, Methacrylamid, Dimethylaminoethylmethacrylat oder einem Derivat von Acrylamid, Methacrylamid, Dimethylaminoethylmethacrylat oder poly *N*-Isopropylacrylamid und Poly-N-isopropylacrylamid-co-allylamin und bevorzugt PNIPAM mit Poly(p-dioxanon) als Hartsegment in Kombination mit z.B. Poly-L-Lactid als strukturgebendes und degradierbares Element ein temperaturabhängiges Quellvermögen in Wasser, das bei einer Temperaturerhöhung von 10 K um 30-50% sinkt. Diese veränderlichen Quellzustände erlauben es nun einen hydrolytischen Abbau von Esterfunktionen aufgrund des temperaturabhängigen Wassergehalts zu steuern.

Die bisher bekannten Lösungen haben aber eine Reihe von Nachteilen:
Die Beeinflussung der Degradation, basierend auf pH, Ionenstärke und/oder Temperaturänderung lassen sich in vivo nur sehr schwer durchführen, da der menschliche Organismus sehr empfindlich auf Änderung dieser Parameter reagiert und nur eine sehr geringe Abweichung vom Normalzustand toleriert. Elektromagnetische Strahlung hingegen kann in niedrigen Energiebereichen Gewebe nur unzureichend penetrieren,
wodurch Polymere in tieferliegenden Gewebsschichten (z.B. auf Stents in Koronargefäßen) nicht erreicht werden können. Dahingegen stellt höherenergetische Strahlung ein grundsätzliches Gefährdungspotential für den Organismus dar, könnte aber zelluläres Gewebe sehr tief durchdringen (Röntgenstrahlen). Elektrische und magnetische Felder zeigen nicht die voran angeführten Einschränkungen, bedürfen aber eines erheblichen apparativen Aufwandes, was den Einsatz dieser Verfahren im medizinischen Bereich erheblich erschwert. Zudem wird eine zeitnahe, automatische Reaktion des Systems dadurch erschwert.

### <hier bitte Seite 4a einfügen>

Aufgabe der Erfindung war es daher, einen implantierbaren Gegenstand anzugeben, der hinsichtlich der Steuerbarkeit seines Degradationsverhaltens für den Explantationsfall verbessert ist.

Diese Aufgabe wird gelöst durch einen implantierbaren Gegenstand, umfassend einen Festkörper (Implantatgrundkörper) darauf angeordnet eine Schicht, umfassend ein Co-Polymer, das ein Aptamer enthält, wobei in Abhängigkeit von einem Bindungsereignis an das Aptamer Bindungen im Co-Polymer gelöst werden können, so dass eine Degradation des Co-Polymers erfolgt.

Ein implantierbarer Gegenstand im Sinne der Erfindung ist dabei ein Gegenstand, der so ausgestaltet ist, dass er in den menschlichen oder tierischen Körper eingebracht und für eine Zeit von wenigstens 5 Tagen in diesem verbleiben kann.

Implantierbare Gegenstände im Sinne der vorliegenden Erfindung sind dabei solche Gegenstände, die in den Körper eingebracht werden um Funktionen zu erfüllen, die nicht durch die aptamerhaltige Schicht allein gewährleistet werden. So ist z.B. eine Funktion eines entsprechend beschichteten Stens primär eine Stützfunktion für ein Gefäß, während die Funktion der aptamerhaltigen Schicht das Gewährleisten einer besseren Explantierbarkeit ist.

In US 2011/0027181 A1 werden Knochenkäfige beschrieben. Im Journal of the American Chemical Society 2008, 130, 6320 wird ein Hydrogel beschrieben. EP2476456 offenbart dauerimplantierbare Elektroden oder Sonden mit einem Regelelement, dessen physikalisch-chemischer Zustand mittels externer Anregung gezielt manipulierbar ist, so dass eine lokale Degradation oder Auflösung des Implants einsetzt und damit eine Explantation erleichtert wird.

Für die erfindungsgemäßen implantierbaren Gegenstände ist es dementsprechend wichtig zu verstehen, dass die aptamerhaltigen Schichten im Sinne der vorliegenden Erfindung immer noch ein von ihnen abweichendes Material umschließen oder zumindest teilweise bedecken. Dieses Material ist oder umfasst zumindest ein festes Material, das mit dem Begriff "Festkörper" bezeichnet ist. Regelmäßig wird es sich dabei um den Hauptbestandteil des Implantates (z.B. im Falle eines Stents die entsprechende Legierung) handeln.

Ein Aptamer im Sinne dieser Erfindung ist ein Polymer aus Ribonucleotiden und/oder Desoxydribonucleotiden sowie deren Analoga wie z. B. Nucleotidanaloga mit Peptidbausteinen (PNA), Nucleotide mit Phosphothioaten, Nucleotidanaloga mit Morpholinobausteinen und LNA (locked nucleic acid). Hier ist eine zusätzliche chemische Bindung zwischen dem 2'O Atom und dem 4'C Atom des Riboserestes innerhalb der RNA Base eingeführt. Dies ermöglicht es, die Ribose in der 3'-endo Konformation zu fixieren, was deren Stabilität gegenüber Endo- und Exonucleasen wesentlich erhöht.

"Aptamere" im Sinne des vorliegenden Textes zeigen die Eigenschaft, unter Konformationsänderung an organische Moleküle zu binden. Diese Affinität kann beispielsweise durch sukzessive Selektionen von verschiedenen Kettenmolekülen bestehend aus den oben genannten Bausteinen massiv erhöht werden (SELEX-Prozess).

Eine bevorzugte Variante eines Aptamers ist ein Spiegelmer. Hierbei wird das Aptamer aus nicht-natürlich vorkommenden, zu den natürlich vorkommenden wenigstens in den Teilbereichen in enatiomeren Nucleotiden gebildet. Hier ist z. B. zu nennen der Austausch der natürlichen D-Ribose gegen die künstliche L-Ribose in der Zuckereinheit des Nucleotides.

Die Größe von Aptameren im Sinne dieser Erfindung ist bevorzugt 10 - 100 und besonders bevorzugt 10-50 Nucleotide bzw. deren Analoga.

Ein Co-Polymer im Sinne dieser Erfindung ist ein Polymer, das neben dem Aptamer noch weitere repetitive Monomeruntereinheiten umfasst. Bevorzugt im Sinne der Erfindung ist ein Block-Co-Polymer.

"Bindungen im Co-Polymer" im Sinne der Erfindung sind kovalente Bindungen, Wasserstoff-Brücken-Bindungen und ionische Wechselwirkungen. Das bedeutet, dass im Sinne der Erfindung ein Co-Polymer aus verschiedenen (Block-)Untereinheiten gebildet werden kann, die untereinander durch Wasserstoffbrückenbindungen verbunden sind. Dies ist zum Beispiel der Fall, wenn die Co-Polymeruntereinheiten jeweils aus einem Aptamer oder einem zu diesem Aptamer komplementären Polyoligonucleotid und einem weiteren Polymer bestehen. Dadurch können über die Wasserstoffbrückenbindungen zwischen dem Aptamer und dem komplementären Strang aus jeweils einer Co-Polymeruntereinheit diese Untereinheiten zusammen zum Co-Polymer gebunden sein.

"Degradation des Co-Polymers" bedeutet im Sinne des vorliegenden Textes, dass die Festigkeit des Verbandes des Gesamt-Polymers geschwächt wird, was im Regelfall durch Bindungslösung erfolgt.

Ein Bindungsereignis im Sinne dieser Erfindung ist das Anlagern wenigstens einer Verbindung an eine zweite Verbindung oder an eine Domäne einer zweiten Verbindung. Bevorzugt ist bei einem solchen Bindungsereignis die Domäne der zweiten Verbindung ein Aptamer. Dabei kann die Anlagerung durch beliebige Arten von Wechselwirkungen erfolgen, bevorzugt sind aber ionische Wechselwirkungen, Wasserstoffbrückenbindungen und kovalente Bindungen.

Eine Schicht, die ein erfindungsgemäß implantierbarer Gegenstand umfasst, ist im Sinne der vorliegenden Erfindung insbesondere ein dreidimensionales Gebilde, das über zwei Hauptflächen (eine Innen- und eine Außenfläche) verfügt (das sind die beiden größten Flächen des Gebildes). Dabei die Wurzel des Flächeninhaltes der Außen- bzw. der Innenfläche jeweils um einen Faktor größer gleich ≥ 10, bevorzugt größer gleich ≥ 20, besonders bevorzugt größer gleich ≥ 100 größer als die mittlere Dicke des Raumes zwischen diesen beiden Flächen. Eine Schicht im Sinne des vorliegenden Textes kann geschlossen sein, das bedeutet, dass sie einen (Raum-)Teil des implantierbaren Gegenstandes vollständig umschließt oder aber sie kann offen sein, so dass kein vollständig geschlossener Raum-Teil des Implantates durch die Schicht begrenzt wird. Bevorzugt im Sinne der vorliegenden Erfindung ist, dass die Schicht geschlossen ist. Ferner kann eine Schicht ihrerseits wieder teilweise oder vollständig beschichtet sein. Dies ist in einigen Fällen ebenfalls bevorzugt. In den meisten Fällen ist es aber bevorzugt, dass eine Aptamer umfassende Schicht wenigstens einen Teil der Oberfläche des implantierbaren Gegenstandes, bevorzugt die gesamte Oberfläche des implantierbaren Gegenstandes bildet.

Bevorzugte Schichtdicken für die aptamerhaltige Schicht des erfindungsgemäßen Gegenstandes sind dabei 0.1 µm - 100 µm, bevorzugt aber 0.5µm - 10 µm und besonders bevorzugt zwischen 1 µm und 5 µm.

In manchen Fällen kann es bevorzugt sein, dass die aptamerhaltige Schicht keine physiologisch wirksamen Substanzen umfasst.

Der Vorteil eines erfindungsgemäß implantierbaren Gegenstandes liegt darin, dass durch eine geeignete Auswahl eines Aptamers gezielt der Stimulus bestimmt werden kann, der die Degradation des Co-Polymers auslösen soll. Insbesondere ist es möglich, rechtzeitig vor einer Explantation des implantierbaren Gegenstandes einen Stimulus zu setzen, der eine Degradation der aptamerhaltigen Schicht bewirkt. Durch diese Degradation würde die Verbindung zwischen dem das Implantat umgebenden Gewebe und dem Implantat geschwächt und idealerweise vollständig gelöst. Auf diese Art lässt sich das Implantat ohne große Schwierigkeiten wieder entfernen, insbesondere ohne dass das körpereigene Gewebe in erhebliche Mitleidenschaft gezogen wird.

Dementsprechend ist im Sinne der Erfindung bevorzugt ein implantierbarer Gegenstand, wobei der Gegenstand so eingerichtet ist, dass im in einen menschlichen oder tierischen Körper implantierten Zustand das Bindungsereignis erfolgt, ausgelöst durch eine systemisch zugeführte Verbindung und/oder unter Beteiligung einer solchen Verbindung. Systemisch hinzugefügte Verbindung in diesem Sinne ist eine Verbindung, die gezielt dem Organismus zugegeben wird, in dem der implantierbare Gegenstand implantiert ist. Die systemisch zugeführte Verbindung ist dabei bevorzugt ein körperfremder Stoff oder ein körpereigener Stoff, der dem Körper in einer Konzentration zugefügt wird, so dass sich im Körper eine überphysiologische Konzentration einstellt.

Bevorzugte systemisch hinzuzufügende Verbindungen sind dabei nicht-natürliche Analoga von körpereigenen Stoffen. Besonders geeignet in diesem Zusammenhang und in vielen Fällen auch bereits bestens untersucht sind dabei Stoffe, die für diagnostische Zwecke eingesetzt werden und dementsprechend auch in größeren Mengen körperverträglich sind. Besonders bevorzugt sind in diesem Zusammenhang drei Stoffklassen zu nennen, nämlich:
Stoffklasse 1 (Halogenierte Mono- und Oligosaccharide und Zuckeralkohole):
   Allgemein wird dabei die OH Gruppe am C2 oder C3 Atom des Zuckermoleküls durch ein Halogenatom (F, Cl, Br) ersetzt.

### Bevorzugte Beispiele:

Glucose: 2-Fluoro-2-deoxy-D-glucose (Kontrastmittel PET Imaging)
Fructose: 2-Fluoro-2-deoxy-D-fructose
Galactose: 2-Fluoro-2-deoxy-D-galactose
Weiterhin bevorzugt ist fluorierte Saccharose, Lactose, Xylitol, Sorbit, Maltit und/oder
Erythrit.

Stoffklasse 2 (Mono- und Oligosaccharide in der L-Form):
Dabei handelt es sich um Zucker, die in der Natur und somit in der Nahrungskette nicht vorkommen. Da es sich um chemisch identische Strukturen mit physikalisch ähnlichen Eigenschaften handelt, können diese Substanzen problemlos auch in größeren Mengen eingenommen werden. Die chiralen Unterschiede der einzelnen Monosaccharide können von Aptameren erkannt werden.

### Bevorzugte Beispiele:

L-Pentosen und L-Hexosen, insbesondere L-Glucose, L-Fructose, L-Ribose, L-Galaktose, L-Mannose, L-Arabinose, L-Xylose oder

Zuckersäuren und Aminozucker, insbesondere L-Glucuronsäure, L-Galacturonsäure, L-Glucosamin.

Stoffklasse 3 Halogenierte essentielle und nicht essentielle Aminosäuren:

### Bevorzugte Beispiele:

4-Fluorophenylalanin und Fluoroethylthyrosin.

Mit aus dem Stand der Technik bekannten Methoden ist es dabei dem Fachmann möglich, Aptamere zu entwickeln, die die für die Erfindung gewünschte Funktion (das Lösen von Bindungen im Co-Polymer nach Reaktion mit dem jeweiligen Stimulus) erfüllen können.

Physiologische Bedingungen (wie auch physiologische Konzentration) sind dabei im Sinne dieses Textes die üblichen physiologischen Konzentrationen von Stoffen, die sich bei artgerechter Ernährung im tierischen oder bevorzugt menschlichen Organismus einstellen. Bevorzugt fallen unter diesen Begriff jedoch keine als Krankheit zu definierenden Stoffwechselzustände und insbesondere lediglich Konzentrationen der oben aufgezählten Stoffklassen 1-3 von ≥ 1.500 ppb, bevorzugt 1-1.200 ppb, weiter bevorzugt 120-1.000 ppb, jeweils bezogen auf das Gesamtgewicht einer oder der den implantierbaren Gegenstand kontaktierenden Körperflüssigkeit.

Die Formulierung, dass "das Bindungsereignis erfolgt *ausgelöst durch eine systemisch zugeführte Verbindung*", bedeutet, dass die betreffende Verbindung nicht direkt an dem Bindungsereignis beteiligt sein muss, sondern nur ursächlich für eine Reaktionskaskade sein muss, die zu dem Bindungsereignis führt.

"Das Bindungsereignis erfolgt *unter Beteiligung einer Verbindung*" bedeutet, dass die Verbindung direkt an dem Bindungsereignis beteiligt ist.

Der Vorteil, dass das Bindungsereignis durch die oben aufgezählten Auslöser ausgelöst wird, liegt darin, dass somit genau bestimmt werden kann, unter welchen Bedingungen oder bei Eintritt welcher physiologischen Situation die Degradation des Co-Polymers erfolgt.

Bevorzugt im Sinne der Erfindung ist ein implantierbarer Gegenstand, wobei das Aptamer durch Bindung an einen zu ihm wenigstens teilweise komplementären Strang an einer Vernetzung einzelner Co-Polymer(unter-)einheiten beteiligt ist und durch das Bindungsereignis die Bindung zu dem wenigstens teilweise komplementären Strang gelöst wird.

Ist eine affine Sequenz als Aptamer bekannt, kann diese Sequenz in einer für den Fachmann geeigneten Weise mit einem Polymer zu einem Co-Polymer kombiniert werden. Außerdem kann eine dem Aptamer wenigstens teilweise komplementäre Sequenz mit dem gleichen Polymer ebenfalls copolymerisiert werden. Auf diese Weise entstehen Co-Polymeruntereinheiten, die sich über das Aptamer und einer wenigstens teilweise dazu komplementärem Sequenz sich miteinander zum Co-Polymer verbinden können.

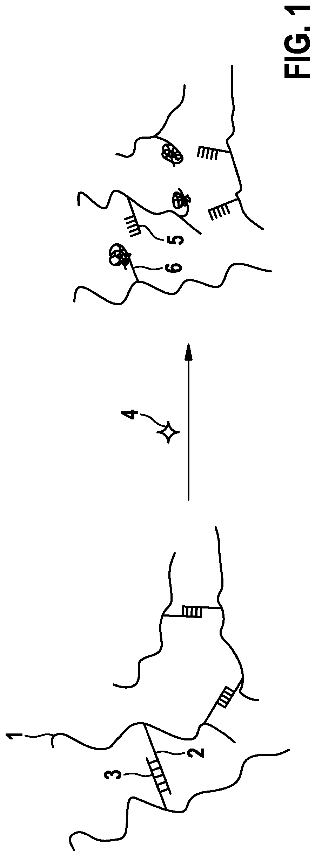

Fig. 1 zeigt schematisch eine solche Bindungssituation, wobei die Bezugszeichen bedeuten:
1 Polymereinheit
2 Aptamer
3 komplementärer Strang zum Aptamer
4 Bindungsereignis auslösendes Molekül (Theophyllin)
5 ungebundene komplementäre Sequenz
6 Aptamer mit Bindung an Theopyllin

Ein Beispiel für die in Fig. 1 beschriebene Situation stellt das Aptamer mit der Sequenz AUACCAUAUGC dar, das eine höhere Affinität zu Theophyllin aufweist als zu seiner Komplementärsequenz UAUGGUAUACG. Wird das Aptamer und seine komplementäre Sequenz jeweils als Seitenkettenfunktion z. B. in lineare Acrylatpolymere eingeführt, so kann eine Co-Polymer-Matrix aufgebaut werden, die selektiv unter Zugabe von Theophyllin degradiert.

Dementsprechend können durch Variation der Polymerblöcke im Co-Polymer gezielt die Verhältnisse so eingestellt werden, dass die Degradation ausschließlich durch Zugabe von Theophyllin ausgelöst wird.

Bevorzugt ist ein erfindungsgemäß implantierbarer Gegenstand, wobei das Aptamer in Wechselwirkung mit einem Ribozym steht oder Teil eines Ribozyms ist und durch das Bindungsereignis die Aktivität des Ribozyms hin zu verstärkter Bindungsspaltung von Bindungen im Co-Polymer verschoben wird.

Als Alternative oder zusätzliche Variante zu der direkten Bindungslösung von Aptameren zu ihren komplementären Sequenzen besteht erfindungsgemäß auch die Möglichkeit, dass das Aptamer ein Bestandteil eines Ribozyms ist.

Ein Ribozym im Sinne dieses Textes ist ein RNA Molekül mit einer genau definierten Tertiärstruktur, welche es erlaubt, eine bestimmte chemische Reaktion zu katalysieren. Dabei kann bevorzugt die chemische Reaktion durch Anwesenheit eines systemisch zugeführten Stimulus um ein Vielfaches beschleunigt werden (allosterische Wechselwirkung). Dabei handelt es sich bei der chemischen Reaktion im Sinne dieses Textes bevorzugt um die Hydrolysereaktion einer Phosphordiesterbindung eines zweiten RNA Moleküls. Dieses zweite RNA Molekül stellt hierbei einen Teil des Co-Polymergrundgerüsts dar.

Ribozyme können so ausgestaltet sein, dass sich ihre enzymatische Aktivität auf die Spaltung von Bindungen innerhalb des Co-Polymeres bezieht. So sind Ribozyme beispielsweise in der Lage, komplementäre RNA-Stränge, die an das Ribozym gebunden sind, zu spalten. Dabei ist das System so ausgestaltbar, dass Bindungen des molekularen Stimulus an die Aptamereinheit die enzymatische Aktivität der ribozymgebundenen Einheit gesteigert wird (bevorzugt um ein Vielfaches). Dadurch wird der an das Ribozym gebundene komplementäre Strang gespalten und abgelöst.

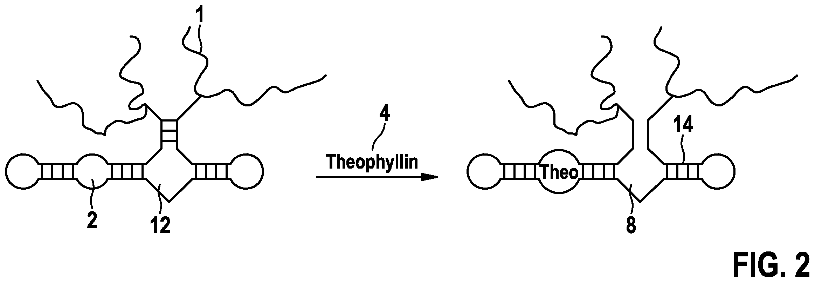

Fig. 2 stellt eine solche Situation dar. Dabei bedeuten die Bezugszeichen:
1 Polymer
2 Aptamer
4 Ereignis auslösendes Molekül
8 Ribozym nach Bindungsereignis
12 Ribozym
14 Strangbruch

Mit diesem bevorzugten System besteht eine weitere Möglichkeit ein erfindungsgemäßes Implantat auszugestalten und für eine Reihe von Anwendungen einzusetzen.

Eine Ausgestaltungsmöglichkeit der Variation mit Ribozymen ist, dass ein allosterisch aktivierbares Ribozym eingesetzt wird. Allosterisch bedeutet dabei, dass das Bindungsereignis an einer anderen Stelle des Ribozyms erfolgt, als am katalytisch aktiven Zentrum.

Eine besondere Ausgestaltung dieser Version ist die enzymatisch aktive RNA-Sequenz (einem sogenannten Hammerhead Ribozym), die eine RNA-Sequenz mit dem Sequenzmotiv UAG (sogenannte IVup Motiv) spalten kann, nachdem ein Bindungsereignis am allosterisch mit dem Ribozym verbundenen Aptamer (chemische Stimulation) durch eine systemisch zugeführte Verbindung im Sinne dieser Erfindung erfolgt ist. Durch dieses Bindungsereignis erhöht sich die katalytische Aktivität des Ribozyms um ein Vielfaches und ermöglicht es so, gezielt durch Zugabe eines chemischen Stimulus das Co-Polymer spezifisch an dem UAG Sequenzmotiv zu spalten (siehe auch hierzu R. Breaker, Engineered allosteric ribozymes as biosensor components).

Bevorzugt ist ein implantierbarer Gegenstand, wobei das Co-Polymer mit einem Wirkstoff beladen ist, der bei Degradation des Co-Polymers verstärkt freigesetzt werden kann. Die Beladung von Polymeren und Co-Polymeren mit Wirkstoffen ist Stand der Technik und zum Beispiel in DE 10 2008 040 786 ausführlich beschrieben.

Für viele Anwendungen bevorzugt ist ein erfindungsgemäßer Gegenstand, wobei das Co-Polymer eine äußere Schutzschicht bildet für unter physiologischen Bedingungen degradierbare Materialien.

Dieser Ansatz eröffnet z. B. die Möglichkeit, zeitlich konstante degradierbare Implantate wie z. B. AMS (Absorbable Metal Stent; Stents bestehend aus einer Magnesiumlegierung, die unter physiologischen Bedingungen abgebaut wird) so lange vor einer Degradation zu schützen, bis durch die Gabe des Bindungsereignis auslösenden Stoffes (Stimulus) die äußere Schutzschicht aus Co-Polymer abgebaut wird, so dass die darunter liegenden Schichten dem unter physiologischen Bedingungen möglichen Abbau zugänglich gemacht werden.

So ist es beispielsweise möglich, den genauen Zeitpunkt zu definieren, zu dem der Abbauprozess eines biologisch degradierbaren Implantates (wie z.B. eines Stents aus einem biologisch degradierbaren Material) beginnen soll.

Für viele Anwendungen ist ein erfindungsgemäßer Gegenstand bevorzugt, bei dem die Explantation durch die durch das Bindungsereignis ausgelöste Degradation des Co-Polymers erleichtert wird.

Ist das erfindungsgemäß einzusetzende Co-Polymer als äußerste Beschichtung eines Implantates, z. B. einer Schrittmacherelektrode, ausgeführt, so ist es vereinfacht, das Implantat wieder zu entfernen, z. B. nach Funktionsverlust wie einem Elektrodenbruch. Durch die systemische Gabe eines das Bindungsereignis auslösenden Stimulus (einer entsprechenden Verbindung) kann das Implantat von dem umschließenden Gewebe einfach dadurch abgelöst werden, dass sich die äußere Beschichtung aus dem Co-Polymer auflöst und so den Kontakt zwischen Implantat (z. B. Elektrode) und Gewebe lockert. Hierdurch wird die Wiederentnahme (Explantation) des Implantates deutlich vereinfacht. Bevorzugte implantierbare Gegenstände sind Gegenstände, ausgewählt aus der Gruppe bestehend aus Herzschrittmacher, Stent, Schrittmacherelektrode, Stimulationselektrode, cerebraler Katheter, Gelenkersatz, Implantat zur Osteosynthese, Dentalimplantat und Implantat der plastischen Chirurgie.

Bevorzugt im Sinne der Erfindung ist es ferner, dass das für den erfindungsgemäßen Gegenstand einzusetzende Co-Polymer neben dem Aptamer (oder einer dem Aptamer komplementären Sequenz) Monomere umfasst, ausgewählt aus der Gruppe bestehend aus Acrylamid, Hydroxyethylmethacrylat, Ethylenglycolmethacrylate, Glucosylethylmethacrylat, Hydroxypropylmethacrylamide, N-isopropylacrylamid, Vinylpyrrolidon, Vinylalcohol, Vinylacetat, Vinylacetat Caprolacton, Hydroxybutyrat, Milchsäure, Lactic-co-gylcolic acid, Ethylenglycol, Propylenglycol.

Der eigentliche Kern der Erfindung ist die Verwendung einer Schicht, umfassend ein Co-Polymer, das ein Aptamer enthält, wobei die Schicht ausgestaltet ist, wie weiter oben beschrieben, zur Verbesserung der Explantierbarkeit eines implantierbaren Gegenstandes.

Der Ansatz durch ganz gezielte Degradierung einer Schicht über die Funktion von Aptameren eine Explantation zu unterstützen, ist hierbei neu. Dabei kommt es auch nicht zwingend darauf an, dass in dem noch nicht implantierten Gegenstand die aptamerhaltige Schicht die äußerste Schicht bildet: Es kann gewünscht sein, dass auf der erfindungsgemäß zu verwendenden aptamerhaltigen Schicht weitere Schichten aufgebracht sind. Diese können z.B. ebenfalls degradierbar sein und so das Einwachsverhalten positiv beeinflussen. Dabei können diese zusätzlichen Schichten weitere Wirkstoffe tragen oder auch nicht. Das gleiche gilt auch selbstverständlich für die aptamerhaltige Schicht, die letztendlich die Explantation vereinfachen soll.

Sofern weitere Schichten auf der die Explantation unterstützenden Schicht aufgebracht sind, so können diese im Rahmen des Einwachsprozesses beispielsweise abgebaut werden, so dass die aptamerhaltige Schicht dann diejenige Schicht ist, die mit dem einwachsenden Gewebe in Berührung kommt. Es ist aber auch grundsätzlich möglich, dass die aptamerhaltige Schicht sich in einem inneren Bereich des Implantates befindet, wenn eine Explantation eingeleitet werden soll. Auch in diesem Fall kann sie ihre erfindungsgemäße Funktion erfüllen, sofern nur gewährleistet ist, dass der Trigger für die Degradierung die Schicht erreichen kann.

### Beispiele:

### Beispiel 1:

Im Folgenden wird ein Beispiel zur Herstellung eines aptamerhaltigen Co-Polymers beschrieben:

### Herstellung funktionelles Aptamer sensitiv auf Theophyllin:

Zur Kopplung der Aptamere an ein Polymer wird an dessen 5' Ende eine Methacrylgruppe eingeführt (Acrydite, Mosaic Technologies, Boston, Mass). Dies ermöglicht die Co-Polymerisation der RNA Sequenz mit z.B. Acrylamid, Hydroxyethylmethacrylat:
Sequenz 1: 5'Acrydite-AAAA AUACCAUAUGC
Sequenz 2: 5'Acrydite-AAAAUAUGGUAUACG

### Bildung Co-Polymer:

Sequenz 1 und 2 wurden zu je 3 mM in 10 mM Tris (pH 8.0), 200mM NaCl und 4% Acrylamid (v/v) gelöst und entgast (Lösung A). Anschließend wurde eine entgaste Mischung aus 0.5 ml H₂O, 0.05 Ammoniumpersulfat und 25 ml Tetraethylmethylendiamin hergestellt (Lösung B). 1.4% (v/v) der Lösung B wurden zu Lösung A hinzugefügt und für 2 min polymerisiert.

### Herstellung Implantatbeschichtung

Der Implantatgrundkörper [z.B. AMS (Stent) oder Schrittmacherelektrode] wird zur Aktivierung unter Feuchtigkeitsausschluss mit Methyl-2-cyanoacrylat besprüht. Anschließend wird der aktivierte Implantatgrundkörper mit der teilpolymerisierten Lösung besprüht und für weitere 10 min auspolymerisiert.

### Beispiel 2:

### Herstellung funktionelles Ribozym sensitiv auf Theophyllin:

Zur Kopplung des Ribozyms an ein Polymer wird an einem der Sequenzen an dessen 5' und 3' Ende eine Methacrylgruppe eingeführt (Acrydite, Mosaic Technologies, Boston, Mass). Dies ermöglicht die Co-Polymerisation der RNA Sequenz mit z.B. Acrylamid, Hydroxyethylmethacrylat:
Sequenz 1: 5'Acrydite-AAAAGCCGUAGGUUGCCCAAA-Acrydite 3'
Sequenz 2: 5' pppGGGCGACCCUGAUGAGUCUGGAUACCAUGCAUGAUGCACCUUGGCAGUC UUACGAAACGGU 3'

### Bildung Co-Polymer:

Sequenz 1 und 2 wurden zu je 3 mM in 10 mM Tris (pH 8.0), 200mM NaCl und 4% Acrylamid (v/v) gelöst und entgast (Lösung A). Anschließend wurde eine entgaste Mischung aus 0.5 ml H₂O, 0.05 Ammoniumpersulfat und 25 ml Tetraethylmethylendiamin hergestellt (Lösung B). 1.4% (v/v) der Lösung B wurden zu Lösung A hinzugefügt und für 2 min polymerisiert.

### Herstellung Implantatbeschichtung

Der Implantatgrundkörper wird zur Aktivierung unter Feuchtigkeitsausschluss mit Methyl-2-cyanoacrylat besprüht. Anschließend wird der aktivierte Implantatgrundkörper mit der teilpolymerisierten Lösung besprüht und für weitere 10 min auspolymerisiert.

### Vorbereitung zur Explantation

Wird die Explantation eines Implantats, hier insbesondere einer Schrittmacherelektrode, notwendig, so kann der Patient im Sinne der Erfindung dadurch zur Explantation vorbereitet werden, dass man dem zum Aufbau der Implantatsbeschichtung eingesetzten Aptamer entsprechend ein Bindungsereignis auslösendes Molekül beispielsweise intravenös zuführt. Das Zuführen dieser Moleküle bewirkt, dass das zum Aufbau der Implantatsbeschichtung verwendete Polymer bevorzugt abgebaut wird. Dazu wird dem Patienten das Bindungsereignis auslösende Molekül in einer Konzentration zugeführt, die über dem der physiologisch nachzuweisenden Menge, aber unter der maximal erlaubten Konzentration liegt.

So muss beispielsweise bei der Explantation einer 40 cm langen Schrittmacherelektrode, welche mit einer 10 µm dicken Beschichtung versehen wurde ein Gesamtvolumen von 5*10⁻⁸ m³ abgebaut werden. Entsprechend ergibt sich eine Gesamtmenge von 150 nmol Aptamer innerhalb der Beschichtung. Um diese vollständig abzubauen sind ebenfalls 150 nmol des Bindungsereignis auslösenden Moleküls notwendig. Da diese entsprechend im Blut verdünnt werden, kann es generell sinnvoll sein, dass die gleiche Konzentration des das Bindungsereignis auslösenden Moleküls im Blut vorhanden ist, wie sie in der Implantatsbeschichtung vorherrscht. In dem zuvor beschriebenen Fall also 3 mM. Im Falle von Glucoseanaloga als das Bindungsereignis auslösendes Molekül liegt man damit in einem Konzentrationsbereich der der physiologisch relevanten Konzentration von D-Glucose entspricht. In vielen Fällen ist es aber bevorzugt, dass die Konzentration des das Bindungsereignis auslösenden Moleküls deutlich niedriger ist, z.B. im Bereich von 1-1.500 ppb. Dann kann eine längere Einwirkzeit erforderlich sein.

Im zuerst vorgeschlagenen Fall werden somit z.B. 50 ml einer 30 mM Lösung von 2-Fluoro-Deoxyglucose in einer physiologischen Kochsalzlösung über einen Zeitraum von 10-20 min. infundiert. Nach einer Inkubationszeit von 2 h kann mit den Explantationsvorbereitungen begonnen werden.

## Patentansprüche

1. Implantierbarer Gegenstand, umfassend einen Festkörper und darauf angeordnet eine Schicht, umfassend ein Co-Polymer, das ein Aptamer enthält, wobei in Abhängigkeit von einem Bindungsereignis an das Aptamer Bindungen im Co-Polymer gelöst werden können, so dass eine Degradation des Co-Polymers erfolgt.

2. Implantierbarer Gegenstand nach Anspruch 1, wobei der Gegenstand so eingerichtet ist, dass im in einen menschlichen oder tierischen Körper implantierten Zustand das Bindungsereignis erfolgt, ausgelöst durch eine systemisch zugeführte Verbindung und/oder unter Beteiligung einer solchen Verbindung.

3. Implantierbarer Gegenstand nach Anspruch 1 oder 2, wobei das Aptamer durch Bindung an einen zu ihm wenigstens teilweise komplementären Strang an einer Vernetzung einzelner Co-Polymereinheiten beteiligt ist und durch das Bindungsereignis die Bindung zu dem wenigstens teilweise komplementären Strang gelöst wird.

4. Implantierbarer Gegenstand nach einem der vorangehenden Ansprüche, wobei das Aptamer in Wechselwirkung mit einem Ribozym steht oder Teil eines Ribozyms ist und durch das Bindungsereignis die Aktivität des Ribozyms hin zu verstärkter Bindungsspaltung von Bindungen im Co-Polymer verschoben wird.

5. Implantierbarer Gegenstand nach einem der vorangehenden Ansprüche, wobei das Co-Polymer mit einem Wirkstoff beladen ist, der bei Degradation des Co-Polymers verstärkt freigesetzt werden kann.

6. Implantierbarer Gegenstand nach einem der vorangehenden Ansprüche, wobei das Co-Polymer eine äußere Schutzschicht bildet für unter physiologischen Bedingungen degradierbare Materialien.

7. Implantierbarer Gegenstand nach einem der vorangehenden Ansprüche, wobei das Bindungsereignis ausgelöst wird durch einen Stoff ausgewählt aus der Gruppe bestehend aus halogenierten Monosacchariden, halogenierten Polyoligosacchariden, halogenierten Zuckeralkoholen, Monosacchariden in L-Form, Oligosacchariden in L-Form und halogenierten Aminosäuren.

8. Implantierbarer Gegenstand nach einem der vorangehenden Ansprüche, wobei das Co-Polymer Monomere umfasst, ausgewählt aus der Gruppe bestehend aus, Acrylamid, Hydroxyethylmethacrylat, Ethylenglycolmethacrylate, Glucosylethylmethacrylat, Hydroxypropylmethacrylamide, N-isopropylacrylamid, Vinylpyrrolidon, Vinylalcohol, Vinylacetat, Vinylacetat Caprolacton, Hydroxybutyrat, Milchsäure, Lactic-co-gylcolic acid, Ethylenglycol, Propylenglycol.

9. Implantierbarer Gegenstand nach einem der vorangehenden Ansprüche, ausgewählt aus der Gruppe bestehend aus Herzschrittmacher, Stent, Schrittmacherelektrode, Stimulationselektrode, cerebraler Katheter, Gelenkersatz, Implantat zur Osteosynthese, Dentalimplantat und Implantat der plastischen Chirurgie.

10. Verwendung einer Schicht, umfassend ein Co-Polymer, das ein Aptamer enthält, wobei die Schicht ausgestaltet ist wie in einem der vorhergehenden Ansprüche beschrieben, zur Verbesserung der Explantierbarkeit eines implantierbaren Gegenstandes.

## Claims

1. An implantable object, comprising a solid body and, arranged thereon, a layer, comprising a copolymer that contains an aptamer, wherein bonds in the copolymer can be broken in accordance with a binding event at the aptamer, such that the copolymer degrades.

2. The implantable object according to claim 1, wherein the object is adapted such that the binding event occurs in the state implanted in a human or animal body and is triggered by a systemically supplied compound and/or with involvement of such a compound.

3. The implantable object according to claim 1 or 2, wherein the aptamer, by binding to a strand at least partly complementary thereto, is involved in a crosslinking of individual copolymer units, and the bond to the at least partly complementary strand is broken by the binding event.

4. The implantable object according to any one of the preceding claims, wherein the aptamer interacts with a ribozyme or is part of a ribozyme and the activity of the ribozyme is shifted toward increased cleaving of bonds in the copolymer as a result of the binding event.

5. The implantable object according to any one of the preceding claims, wherein the copolymer is charged with an active ingredient, which can be released in an intensified manner in the event of degradation of the copolymer.

6. The implantable object according to any one of the preceding claims, wherein the copolymer forms an outer protective layer for materials that are degradable under physiological conditions.

7. The implantable object according to any one of the preceding claims, wherein the binding event is triggered by a substance selected from the group consisting of halogenated monosaccharides, halogenated polyoligosaccharides, halogenated sugar alcohols, L-monosaccharides, L-oligosaccharides and halogenated amino acids.

8. The implantable object according to any one of the preceding claims, wherein the copolymer comprises monomers, selected from the group consisting of acrylamide, hydroxyethyl methacrylate, ethylene glycol methacrylates, glucosyl ethyl methacrylate, hydroxypropyl methacrylamides, N-isopropylacrylamide, vinylpyrrolidone, vinyl alcohol, vinyl acetate, vinyl acetate caprolactone, hydroxybutyrate, lactic acid, lactic-co-glycolic acid, ethylene glycol, propylene glycol.

9. The implantable object according to any one of the preceding claims, selected from the group consisting of heart pacemakers, stents, pacemaker electrodes, stimulation electrodes, cerebral catheters, joint replacements, implants for osteosynthesis, dental implants and plastic surgery implants.

10. Use of a layer, comprising a copolymer that contains an aptamer, wherein the layer is designed as described in any one of the preceding claims, for improving the explantability of an implantable object.

## Revendications

1. Objet implantable comprenant un corps solide et, agencée au-dessus, une couche comprenant un copolymère, qui contient un aptamère, où, en fonction de l'établissement d'une liaison à l'aptamère, des liaisons peuvent être libérées dans le copolymère de sorte qu'un dégradation du copolymère se produit.

2. Objet implantable selon la revendication 1, où l'objet est agencé de telle manière que, dans l'état implanté dans un corps humain ou animal, l'établissement de la liaison a lieu, déclenché par une liaison amenée systématiquement, et/ou moyennant l'implication d'une telle liaison.

3. Objet implantable selon la revendication 1 ou 2, où l'aptamère est impliqué par la liaison au niveau d'un segment au moins partiellement complémentaire à celui-ci à une réticulation d'unités de copolymère individuelles et la liaison vers le segment au moins partiellement complémentaire est libérée par l'établissement de la liaison.

4. Objet implantable selon l'une des revendications précédentes, où l'aptamère est en interaction avec un ribozyme ou une partie d'un ribozyme et, par l'établissement de la liaison, l'activité du ribozyme est déplacée vers une scission renforcée de la liaison de liaisons dans le copolymère.

5. Objet implantable selon l'une des revendications précédentes, où le copolymère est chargé avec une matière active qui peut être libérée plus fortement lors de la dégradation du copolymère.

6. Objet implantable selon l'une des revendications précédentes, où le copolymère forme une couche de protection externe pour des matériaux dégradables dans des conditions physiologiques.

7. Objet implantable selon l'une des revendications précédentes, où l'établissement de la liaison est déclenché par une substance choisie dans le groupe constitué de monosaccharides halogénés, de poly oligosaccharides halogénés, d'alcools de sucres halogénés, de monosaccharides en forme de L, d'oligosaccharides en forme de L et d'acides aminés halogénés.

8. Objet implantable selon l'une des revendications précédentes, où le copolymère comprend des monomères choisis dans le groupe constitué de l'acrylamide, de l'hydroxy éthyl méthacrylate, des éthylène glycol méthacrylates, du glucosyl éthyl méthacrylate, des hydroxy propyl méthacrylamides, du N-isopropyl acrylamide, de la vinylpyrrolidone, de l'alcool vinylique, de l'acétate de vinyle, du caprolactone d'acétate de vinyle, de l'hydroxy butyrate, de l'acide lactique, de l'acide lactique-co-glycolique, de l'éthylène glycol, du propylène glycol.

9. Objet implantable selon l'une des revendications précédentes, choisi dans le groupe constitué des stimulateurs cardiaques, des stents, des électrodes de stimulateurs cardiaques, des électrodes de stimulation, des cathéters cérébraux, des ersatz d'articulations, d'un implant pour l'ostéosynthèse, d'un implant dental et d'un implant de chirurgie plastique.

10. Utilisation d'une couche, comprenant un copolymère qui contient un aptamère, où la couche est conçue de manière décrite selon l'une des revendications précédentes pour l'amélioration de l'implantation d'un objet implantable.
